# NEUE EUROPÄISCHE PATENTSCHRIFT

(11) **EP 1 791 516 B2**
(45) Veröffentlichungstag und Bekanntmachung des Hinweises auf die Entscheidung über den Einspruch: **07.08.2013**
(45) Hinweis auf die Patenterteilung: 24.03.2010
(21) Anmeldenummer: 04804190.9
(22) Anmeldetag: 22.12.2004
(51) Int. Cl.: A61K 8/60, A61Q 5/08, A61Q 5/10

(54) **VERWENDUNG VON ALKYL-OLIGOGLYKOSIDCARBOXYLATEN IN MITTELN ZUR BEHANDLUNG KERATINISCHER FASERN**
USE OF ALKYL-OLIGOGLYCOSIDE CARBOXYLATES IN AGENTS FOR TREATING KERATIN FIBRES
UTILISATION DE CARBOXYLATES D'OLIGOGLUCOSIDES D'ALKYLE DANS DES PRODUITS DESTINES AU TRAITEMENT DE FIBRES KERATINIQUES

(30) Priorität: 05.06.2004 DE 102004027589
(43) Veröffentlichungstag der Anmeldung: 06.06.2007
(73) Patentinhaber: Henkel AG & Co. KGaA, 40589 Düsseldorf (DE)
(72) Erfinder: SEILER, Martina, 47228 Duisburg (DE); HOLLENBERG, Detlef, 40699 Erkrath (DE); PAULI, Kristin c/o Dirk Miehlich, 42119 Wuppertal (DE); KRAH, Stephanie, 40599 Düsseldorf (DE)
(86) Internationale Anmeldenummer: PCT/EP2004/014594
(87) Internationale Veröffentlichungsnummer: WO 2005/120442

(56) Entgegenhaltungen:
- WO-A-91/15192
- WO-A-94/26857
- DE-A1- 19 501 185
- US-A1- 2003 061 668

## Beschreibung

Die Erfindung betrifft die Verwendung von spezifischen Alkyl-Oligoglykosidderivaten in Oxidationsmittel-haltigen Mitteln zur Behandlung keratinischer Fasern, derartige Mittel sowie Verfahren zur oxidativen Färbung keratinischer Fasern.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle.

Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluss von Oxidationsmitteln untereinander oder unter Kupplung mit einer oder mehreren Kupplerkomponenten die eigentlichen Farbstoffe aus. Die Oxidationsfärbemittel zeichnen sich durch hervorragende, lang anhaltende Färbeergebnisse aus.

Haarfärbemittel werden überwiegend in Form von Cremes angeboten, welche die färbenden Komponenten, z.B. die Farbstoffvorprodukte im Falle von Oxidationshaarfärbemitteln oder die Farbstoffe im Fall von direktziehenden Färbemitteln, enthalten. Im Fall von Oxidationshaarfärbemitteln wird die Färbecreme vor der Anwendung mit einer H₂O₂-haltigen Emulsion (Entwickleremulsion) vermischt.

Üblicherweise enthalten die Färbecremes als sogenannte Konsistenzgeber (Fettphase) Fettalkohole, Fettalkoholethoxylate, Fettsäuren oder deren Ester. Weitere Bestandteile sind neben den Farbstoffen nichtionische, anionische und/oder amphotere Tenside, Neutralisationsmittel, Parfümöle, Antioxidantien sowie Pflegekomponenten wie Silikonöle, Proteinhydrolysate, kationische Polymere etc.

Im Fall für den Einsatz als Haarfärbecreme sollte die Cremegrundlage beziehungsweise die fertige Färbecreme eine ausreichend hohe Viskosität und ein geeignetes Fließverhalten nach dem Vermischen mit einer Entwickleremulsion aufweisen, so dass die Mischung leicht auf die zu färbenden Fasern appliziert werden kann und nicht heruntertropft. Ferner sollte die Creme eine weitgehende Viskositätskonstanz im Temperaturbereich zwischen 20°C und 45°C haben, so dass auch beim Erwärmen der Creme auf Hauttemperatur (ca. 32°C) kein Abtropfen erfolgt und auch die Emulsion bei höheren Lagertemperaturen stabil bleibt.

Ferner werden häufig aufhellende Verfahren, die sogenannten Blondierverfahren, angewendet. Die Grundlagen der Blondierverfahren sind dem Fachmann bekannt und in einschlägigen Monographien, z.B. von K. Schrader, Grundlagen und Rezepturen der Kosmetika, 2. Auflage, 1989, Dr. Alfred Hüthig Verlag, Heidelberg, oder W. Umbach (Hrg.), Kosmetik, 2. Auflage, 1995, Georg Thieme Verlag, Stuttgart, New York, zusammenfassend beschrieben.

Zum Blondieren menschlicher Haare - insbesondere für die Strähnchenapplikation - werden üblicherweise feste oder pastenförmige Zubereitungen mit festen Oxidationsmitteln unmittelbar vor der Anwendung mit einer verdünnten Wasserstoffperoxidlösung vermischt. Diese Mischung wird dann auf das Haar aufgebracht und nach einer bestimmten Einwirkzeit wieder ausgespült. Die genannten Zubereitungen, die im Rahmen der Blondierung vor der Anwendung üblicherweise mit einer Wasserstoffperoxidlösung vermischt werden, werden im Weiteren als "Blondiermittel" bezeichnet. Die Mischungen werden als "anwendungsfertige Blondiermittel" bezeichnet.

Durch den erforderlichen Gehalt an Oxidationsmitteln strapazieren oxidative Verfahren die keratinhaltigen Fasern. In einigen Fällen kann es sogar, insbesondere bei den Blondierverfahren, zu nachhaltigen Schädigungen der Fasern kommen. Es hat daher in der Vergangenheit nicht an Versuchen gefehlt, den negativen Einfluss der Oxidationsmittel auf die Fasern zu verringern. Eine Entwicklungsrichtung ist die Optimierung der in den Färbeverfahren eingesetzten Farbstoffe. So wurden beispielsweise Farbstoffe entwickelt, die bereits durch Luftsauerstoff oxidiert werden können und daher den Einsatz weiterer Oxidationsmittel überflüssig machen. Eine weitere Entwicklungsrichtung ist der Einsatz von Enzymen oder Metallsalzen als Oxidationskatalysatoren, um auf diese Weise den erforderlichen Gehalt an Oxidationsmittel zu senken. Ein Nachteil dieser Färbeverfahren ist allerdings der Umstand, dass die natürlichen Farbpigmente der Haare nicht aufgehellt werden. Das hat zur Folge, dass nur Nuancen zugänglich sind, die dunkler sind als die ursprüngliche Haarfarbe.

Bei der Anwendung werden die Blondierungs- oder Färbecremes üblicherweise als ZweiKomponenten-Produkte mit einer wässrigen, sauer eingestellten Formulierung eines Oxidationsmittels gemischt. Die Creme enthält Tenside, die eine intensive Vermischung der beiden Komponenten erlauben. Sowohl Mittel zur oxidativen Färbung von Haaren als auch Mittel zur Blondierung von Haaren strapazieren nicht nur die Haare an sich, sondern reizen in der Regel auch die Kopfhaut. Dieser Effekt ist insbesondere bei Blondierungsmitteln ausgeprägt. Es wurde daher stets nach Verbindungen gesucht, die die Hautreizung in den Mitteln verringern können.

Aufgabe der vorliegenden Erfindung ist die Bereitstellung von tensidischen Komponenten für Mittel zur oxidativen Behandlung keratinischer Fasern, insbesondere zur oxidativen Haarbehandlung, die die Hautreizwirkung der Mittel vermindern. In Mitteln zur oxidativen Färbung von Haaren soll vorzugsweise die Farbstabilität der Ausfärbung verbessert werden.

Die Aufgaben werden erfindungsgemäß gelöst durch Verwendung von Alkyl--Oligoglykosidcarboxylaten, die sich von Alkyl-oligoglykosiden der allgemeinen Formel (I) ableiten,

R-O-(G)ₚ (I)

mit der Bedeutung
- R: C₆₋₂₂-Alkyl,
- G: Glykosideinheit, die sich von einem Zucker mit 5 oder 6 Kohlenstoffatomen ableitet,
- p: Zahl von 1 bis 10,
in Oxidationsmittel-haltigen Mitteln zur Behandlung keratinischer Fasern, insbesondere zur Haarbehandlung, gemäß Anspruch 1

Es wurde erfindungsgemäß gefunden, dass die Verwendung von Alkyl-Oligoglykosidcarboxylaten in Oxidationsmittel-haltigen Mitteln zur Behandlung keratinischer Fasern, insbesondere in Mitteln zur Haarbehandlung zu einer Verminderung der Hautreizung führt. Ferner wurde gefunden, dass bei Anwendung von Mitteln zur oxidativen Färbung von Haaren die Farbstabilität der Ausfärbung durch Mitverwendung der Alkyl-Oligoglykosidcarboxylate verbessert wird. Insbesondere wird diese Wirkung bei Anwendung auf strapaziertem Haar erreicht. Auch die Waschechtheit von gefärbtem strapaziertem Haar wird verbessert.

Unter keratinhaltigen Fasern sind Wolle, Pelze, Federn und insbesondere menschliche Haare zu verstehen.

Unter oxidativen Prozessen werden erfindungsgemäß alle Verfahren verstanden, bei denen die Fasern mit einem Oxidationsmittel behandelt werden. Beispiele für derartige Verfahren stellen die Blondierung, die oxidative Färbung sowie die Fixierung im Rahmen einer Dauerwellbehandlung dar. Unter einem Oxidationsmittel wird erfindungsgemäß kein Luftsauerstoff verstanden.

Erfindungsgemäß werden bevorzugt Alkyloligoglykosidcarboxylate insbesondere Alkylglykosidcarboxylate eingesetzt.

In den Alkyl-Oligoglykosiden ist vorzugsweise in mindestens einem der Reste G mindestens eine Hydroxylgruppe durch -O-C₁₋₁₂-Akylen-COOM mit M H, Alkalimetall, NH₄ ersetzt.

Dabei wird besonders bevorzugt ein Alkyloligoglykosid-Carboxylat eingesetzt, in dem -O-C₁₋₁₂-Alkylen-COOM -O(CH₂-)ₙCOOM mit M H, Na oder K und n = 1 bis 3 bedeutet. Besonders bevorzugt ist der Rest O-CH₂-COONa.

Besonders bevorzugt wird ein Alkyloligoglykosidcarboxylat eingesetzt, in dem der Alkylrest ein Laurylrest ist. Speziell bevorzugt ist ein Laurylglucosidcarboxylat, wie es als Plantapon^{®} LGC und Plantapon^{®} LGC sorb von Cognis Deutschland erhältlich ist.

In den Alkylglykosiden der allgemeinen Formel (I) leiten sich die Glykosid-Einheiten G vorzugsweise von Aldosen bzw. Ketosen ab.

Vorzugsweise werden wegen der besseren Reaktionsfähigkeit die reduzierend wirkenden Saccharide, die Aldosen, verwendet. Unter den Aldosen kommt wegen ihrer leichten Zugänglichkeit und technischen Verfügbarkeit insbesondere die Glucose in Betracht. Die als Ausgangsstoffe besonders bevorzugt eingesetzten Alkylgykoside sind daher die Alkylglucoside.

Die Indexzahl p in der allgemeinen Formel (I) gibt den Oligomerisierungsgrad, d.h. die Verteilung von Mono- und Oligoglykosiden an und steht für eine Zahl zwischen 1 und 10. Während p in einer gegebenen Verbindung stets ganzzahlig sein muss und hier vor allem die Werte p = 1 bis 6 annehmen kann, ist der Wert p für ein bestimmtes Alkylglykosid eine analytische ermittelte rechnerische Größe, die meistens eine gebrochene Zahl darstellt. Vorzugsweise werden Alkylglykoside mit einem mittleren Oligomerisierungsgrad p von 1,1 bis 3,0 verwendet. Besonders bevorzugt sind solche Alkylglykosid, deren Oligomerisierungsgrad kleiner als 1,5 ist und insbesondere zwischen 1,1, und 1,4 liegt.

Der Alkylrest R leitet sich von primären Alkoholen mit 6 bis 22, vorzugsweise 12 bis 18 Kohlenstoffatomen ab. Typische Beispiele sind Capronalkohol, Caprylalkohol, Caprinalkohol, Laurylalkohol, Myristylalkohol, Cetylalkohol, Stearylalkohol und Behenylakohol sowie technische Fraktionen, die neben den genannten gesättigten Alkoholen auch Anteile an ungesättigten Alkoholen enthalten können und die auf Basis von natürlichen Fetten und Ölen, beispielsweise Palmöl, Palmkernöl, Kokosöl oder Rindertalg gewonnen werden. Der Einsatz von technischem Kokosalkohol ist hierbei besonders bevorzugt.

Neben den genannten Fettalkoholen können sich die Alkylglykoside auch von synthetischen primären Alkoholen mit 6 bis 22 Kohlenstoffatomen, insbesondere den sogenannten Oxoalkoholen ableiten, die einen Anteil von 5 bis 40 Gew.-% verzweigter Isomeren aufweisen.

Besonders bevorzugte Alkylreste sind solche mit 8/10, 12/14, 8 bis 16, 12 bis 16 oder 16 bis 18 C-Atomen. Mischungen der Alkylreste ergeben sich bei einer Herstellung ausgehend von natürlichen Fetten und Ölen oder Mineralölen.

Verfahren zur Herstellung dieser Alkylglykoside sind beispielsweise in den amerikanischen Patentschriften US 3,547,828 und US 3,839,318 sowie der deutschen Patentanmeldung DE-A-37 23 826 beschrieben. Für Alkylpolyglykoside an sich kann beispielsweise auf die DE-A-100 27 975, DE-A-101 38 094 und DE-A-100 31 014 verwiesen werden.

Für eine weitere Diskussion der Alkyloligo/polyglykoside (APG) kann auf WO 03/013450 verwiesen werden.

Die Herstellung der erfindungsgemäß eingesetzten Alkyl-Oligoglykosidcarboxylate, kann nach bekannten Verfahren erfolgen. Die Herstellung der Carboxylate erfolgt beispielsweise durch Umsetzung der Alkyloligoglykoside mit Salzen von Chlorcarbonsäuren in Gegenwart von Basen. Beispielsweise kann mit 2-Chloressigsäure-Natriumsalz in Gegenwart von NaOH umgesetzt werden. Bei der Umsetzung können sowohl die Hydroxylgruppen im Ring wie auch die -CH₂-OH-Gruppe umgesetzt werden. Der Umsetzungsgrad ist u.a. abhängig von der Stöchiometrie der Einsatzprodukte. Vorzugsweise werden die Alkyloligoglykoside zumindest an der -CH₂-OH-Gruppe umgesetzt, wobei optional ein Mittel eine oder mehreren der am Ring befindlichen Hydroxylgruppen umgesetzt werden können.

Weitere Hydroxylgruppen können beispielsweise auch verethert sein.

Die Herstellung der Isethionate ist beispielsweise in der WO 94/26857 beschrieben. Dort ist ferner angegeben, dass die Produkte zur Haar- und Körperpflege eingesetzt werden können. Es werden insbesondere wässrige Detergenzgemische beschrieben, die Alkyloligoglykosidisethionate und beispielsweise weitere anionische Tenside enthalten.

Die Herstellung der Sulfate ist beispielsweise in der WO 93/10208 und WO 91/15192 beschrieben. In letzterer Schrift sind auch Gemische der APG-Sulfate mit u.a. Alkylsulfaten oder Alkylethersulfaten sowie weiteren Inhaltsstoffen beschrieben. Es ist angegeben, dass die Tensidmischungen in Produkten eingesetzt werden können, die zum Waschen, Färben, Wellen oder zur Spülung von Haaren dienen.

Die Herstellung der Sulfate kann zudem wie in EP-A-0 186 242 beschrieben erfolgen. Beispielsweise kann das entsprechende Alkylglykosid mit gasförmigem Schwefeltrioxid oder mit Schwefelsäure, gefolgt von Neutralisierung, umgesetzt werden.

Weiterhin kann auf die DE-A-195 00 780 hingewiesen werden, in der kosmetische und pharmazeutische Zubereitungen beschrieben sind, die Alkyloligoglykosidsulfate enthalten.

US 2003/061668 beschreibt Haarfärbemittel, die Alkyloligoglycosidsulfate enthalten können.

In der DE-A-195 01 185 sind Detergenzgemische aus Alkyloligoglykosidsulfaten und Alkyletherphosphaten beschrieben, die beispielsweise in Haarspülungen, Haarfärbemitteln oder Haarwellmitteln eingesetzt werden können.

Die erfindungsgemäß eingesetzten Alkyl-Oligoglykosidcarboxylate, - werden insbesondere in Mitteln zur oxidativen Färbung von Haaren insbesondere in Kombination mit Wasserstoffperoxid und/oder dessen festen Anlagerungsprodukten verwendet. Gemäß einer Ausführungsform werden die Mittel aus einer W/O- und/oder O/W-Emulsion, enthaltend die Alkyl-Oligoglykosidcarboxylate, wie sie vorstehend beschrieben sind, und einer Wasserstoffperoxid-Lösung unmittelbar vor der Anwendung zur oxidativen Färbung von Haaren gemischt. Diese gebrauchsfertigen Mittel enthalten mindestens ein Alkyl-Oligoglykosidecarboxylat vorzugsweise in einer Menge von 0,2 bis 15 Gew.-%, besonders bevorzugt 0,5 bis 10 Gew.-%. In anwendungsfertigen Mitteln zur Blondierung liegen vorzugsweise 0,5 bis 10,5 Gew.-%, besonders bevorzugt 0,5 bis 8 Gew.-%, insbesondere 1 bis 8 Gew.-% der Glykosidverbindungen vor. In anwendungsfertigen Mitteln zur oxidativen Färbung von Haaren liegen vorzugsweise 0,2 bis 6, besonders bevorzugt 0,5 bis 3, insbesondere 0,5 bis 2,1 Gew.-% der Glykosidverbindungen vor.

Die eingesetzten Alkyl-Oligoglycosidcarboxylate können in diesen Mitteln die üblichen anionischen Tenside ganz oder teilweise ersetzen. Damit können die Alkyl-Oligoglycosidcarboxylate als alleiniges anionisches Tensid in den Mitteln eingesetzt werden, oder es können Gemische mit üblichen weiteren anionischen Tensiden eingesetzt werden. Diese übliche anionischen Tenside sind an späterer Stelle näher erläutert. Beispielsweise können die Alkyl-Oligoglycosidcarboxylate und weitere anionische Tenside in einem Gewichtsverhältnis im Bereich von 1 : 0,002 bis 3,5 : 1, besonders bevorzugt 1 : 0,5 bis 1 : 2, insbesondere 1 : 0,75 bis 1 : 1,5 vorliegen. Bevorzugte weitere anionische Tenside sind insbesondere Alkylsulfate oder Alkylethersulfate, speziell Alkylethersulfate wie Laurethsulfat, das beispielsweise als Texapon^{®} NSO von Cognis Deutschland erhältlich ist.

Insbesondere ist es erfindungsgemäß bevorzugt, in einer üblichen Cremegrundlage, die Alkylsulfate, speziell Laurethsulfat, als anionische Tenside enthalten, die Alkylsulfate ganz oder teilweise durch die erfindungsgemäß eingesetzten Glykosidverbindungen zu ersetzen. Hierdurch wird insbesondere in Mitteln zur Blondierung unter sonst gleichen Bedingungen bei der Anwendung bei mindestens gleicher Aufhellungsleistung eine deutlich verringerte Hautreizung bewirkt. In Kombination mit Boosterpulvern, die zur Ultrablondierung eingesetzt werden, wird ein deutlich geringeres Aufschäumverhalten beim Mischen und nach kurzer Standzeit sowie während der Anwendung beobachtet im Vergleich zum Einsatz von Mitteln, die nur Laurethsulfat allein enthalten. Hierdurch kann die Sicherheit des Produktes deutlich verbessert werden, da das Produkt besser handhabbar ist.

In Mitteln zur oxidativen Färbung von Haaren wird insbesondere beim Ersetzen eines Teils oder des ganzen herkömmlichen anionischen Tensids wie Alkylethersulfates, insbesondere Laurethsulfates durch die erfindungsgemäß eingesetzte Alkyl-Oligoglycosidcarboxylate der Formel (I) bei der Ausfärbung und nachfolgend Untersuchung von Colorationen eine deutlich erhöhte Farbstabilität und damit Farbechtheit festgestellt.

Derartige Mittel zur oxidativen Färbung von Haaren enthalten in der Regel mindestens ein Farbstoffvorprodukt vom Typ der Entwickler sowie häufig ebenfalls ein Farbstoffvorprodukt vom Typ der Kuppler. Diese und weitere Inhaltsstoffe wie viskositätsgebende Fettalkohole als Basis neben weiteren Inhaltsstoffen wie Tensiden, Komplexierungsmitteln, Alkalisierungsmitteln wie Ammoniak und Monoethanolamin, Emulgatoren, Puffern, Salzen, pflegenden Rohstoffen und Wasserglas sind nachstehend näher erläutert.

Die erfindungsgemäßen Mittel enthalten üblicherweise als wichtiges Oxidationsmittel Wasserstoffperoxid oder eine feste Anlagerungsverbindung von Wasserstoffperoxid an anorganische oder organische Verbindungen, wie beispielsweise Natriumperborat, Magnesiumpercarbonat, Natriumpercarbonat, Natriumpercarbamid, Polyvinylpyrrolidon x n H₂O₂, Harnstoffperoxid und Melaminperoxid. Die erfindungsgemäßen Mittel können zusätzlich als weiteres Oxidationsmittel so genannte "Booster"-Komponenten enthalten. Dies sind in der Regel feste Peroxoverbindungen, die keine Anlagerungsprodukte von Wasserstoffperoxid an andere Komponenten darstellen. Die Auswahl dieser Peroxoverbindung unterliegt prinzipiell keinen Beschränkungen; übliche, dem Fachmann bekannte Peroxoverbindungen sind beispielsweise Ammoniumperoxidisulfat, Kaliumperoxidisulfat, Natriumperoxidisulfat, Ammoniumpersulfat, Kaliumpersulfat, Natriumpersulfat, Kaliumperoxidiphosphat und Peroxide wie Bariumperoxid und Magnesiumperoxid. Unter diesen Peroxoverbindungen, die auch in Kombination eingesetzt werden können, sind erfindungsgemäß die anorganischen Verbindungen bevorzugt. Besonders bevorzugt sind die Peroxidisulfate, insbesondere Ammoniumperoxidisulfat. Besonders bei der Verwendung von "Boostern", die sich leicht in höheren Konzentrationen in den fertigen Zubereitungen lösen, wie beispielsweise Ammoniumperoxidisulfat, ist aber die Gefahr einer verstärkten Haarschädigung und Kopfhautreizung gegeben.

In einer bevorzugten Ausführungsform der vorliegenden Erfindung ist der oxidative Prozess eine oxidative Haarfärbung. Oxidative Haarfärbemittel enthalten als Vorprodukte für Oxidationsfarbstoffe so genannte Entwickler- und Kupplerkomponenten. Die Entwicklerkomponenten bilden unter dem Einfluss von Oxidationsmitteln, gegebenenfalls mit Hilfe spezieller Enzyme oder Metallionen als Katalysator, untereinander oder unter Kupplung mit einer oder mehreren Kupplerkomponenten die eigentlichen Farbstoffe aus.

Erfindungsgemäß bevorzugte Entwicklerkomponenten sind p-Phenylendiamin, p-Toluylendiamin, p-Aminophenol, o-Aminophenol, 1-(2'-Hydroxyethyl)-2,5-diaminobenzol, N,N-Bis-(2-hydroxy-ethyl)-p-phenylendiamin, 2-(2,5- Diaminophenoxy)-ethanol, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 2-Hydroxymethylamino-4-amino-phenol, Bis-(4-aminophenyl)amin, 4-Amino-3-fluorphenol, 2-Aminomethyl-4-aminophenol, 2-Hydroxymethyl-4-aminophenol, 4-Amino-2-((diethylamino)-methyl)-phenol, Bis-(2-hydroxy-5-aminophenyl)-methan, 1,4-Bis-(4-aminophenyl)-diazacycloheptan, 1,3-Bis(N(2-hydroxyethyl)-N(4-aminophenylamino))-2-propanol, 4-Amino-2-(2-hydroxyethoxy)-phenol, 1,10-Bis-(2,5-diaminophenyl)-1,4,7,10-tetraoxadecan sowie 4,5-Diaminopyrazol-Derivate nach EP 0 740 931 B1 bzw. WO 94/08970 A1 wie z.B. 4,5-Diamino-1-(2'-hydroxyethyl)-pyrazol.

Erfindungsgemäß besonders bevorzugt sind die Entwicklerkomponenten p-Phenylendiamin, p-Toluylendiamin, p-Aminophenol, 1-(2'-Hydroxyethyl)-2,5-diaminobenzol, N,N-Bis-(2-hydroxy-ethyl)-p-phenylendiamin, 4-Amino-3-methylphenol, 4-Amino-2-((diethylamino)-methyl)-phenol, 2-Aminomethyl-4-aminophenol, 2,4,5,6-Tetraaminopyrimidin, 2-Hydroxy-4,5,6-triaminopyrimidin, 4-Hydroxy-2,5,6-triaminopyrimidin und 4,5-Diamino-1-(2'-hydroxyethyl)-pyrazol.

Erfindungsgemäß bevorzugte Kupplerkomponenten sind
- m-Aminophenol und dessen Derivate wie beispielsweise 5-Amino-2-methylphenol, 3-Amino-2-chlor-6-methylphenol, 2-Hydroxy-4-aminophenoxyethanol, 2,6-Dimethyl-3-aminophenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-(Ethylamino)-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2,4-diaminophenoxy)-propan, 1-Methoxy-2-amino-4-(2'-hydroxyethylamino)benzol, 1,3-Bis-(2,4-diaminophenyl)-propan, 2,6-Bis-(2-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol und
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol.

Erfindungsgemäß besonders bevorzugte Kupplerkomponenten sind 1-Naphthol, 1,5-, 2,7-und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Ferner können die oxidativen Färbemittel zur weiteren Nuancierung verschiedene direktziehende Farbstoffe enthalten.

Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, HC Orange 1, Disperse Orange 3, HC Red 1, HC Red 3, HC Red 13, HC Red BN, Basic Red 76, HC Blue 2, HC Blue 12, Disperse Blue 3, Basic Blue 7, Basic Blue 26, Basic Blue 99, HC Violet 1, Disperse Violet 1, Disperse Violet 4, Basic Violet 2, Basic Violet 14, Acid Violet 43, Disperse Black 9, Acid Black 52, Basic Brown 16 und Basic Brown 17 bekannten Verbindungen sowie 1,4-Bis-(β-hydroxyethyl)-amino-2-nitrobenzol, 3-Nitro-4-(β-hydroxyethyl)-aminophenol, 4-Amino-2-nitrodiphenylamin-2'-carbonsäure, 6-Nitro-1,2,3,4-tetrahydrochinoxalin, 2-Hydroxy-1,4-naphthochinon, Hydroxyethyl-2-nitro-toluidin, Pikraminsäure, 2-Amino-6-chloro-4-nitrophenol, 4-Ethylamino-3-nitrobenzoesäure und 2-Chloro-6-ethylamino-1-hydroxy-4-nitrobenzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die Färbezubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Weitere in Färbemitteln enthaltene Farbstoffkomponenten können auch Indole und Indoline, sowie deren physiologisch verträgliche Salze, sein. Bevorzugte Beispiele sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol. Weiterhin bevorzugt sind 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure, 6-Hydroxyindolin, 6-Aminoindolin und 4-Aminoindolin.

Die Indolin- und Indol-Derivate können sowohl als freie Basen als auch in Form ihrer physiologisch verträglichen Salze mit anorganischen oder organischen Säuren, z. B. der Hydrochloride, der Sulfate und Hydrobromide, eingesetzt werden.

Bei der Verwendung von Farbstoff-Vorstufen vom Indolin- oder Indol-Typ kann es bevorzugt sein, diese zusammen mit mindestens einer Aminosäure und/oder mindestens einem Oligopeptid einzusetzen. Bevorzugte Aminosäuren sind Aminocarbonsäuren, insbesondere α-Aminocarbonsäuren und ω-Aminocarbonsäuren. Unter den α-Aminocarbonsäuren sind wiederum Arginin, Lysin, Ornithin und Histidin besonders bevorzugt. Eine ganz besonders bevorzugte Aminosäure ist Arginin, insbesondere in freier Form, aber auch als Hydrochlorid eingesetzt.

Oxidative Haarfärbemittel werden üblicherweise schwach sauer bis alkalisch, d. h. auf pH-Werte im Bereich von etwa 5 bis 11, eingestellt. Zu diesem Zweck enthalten die Färbemittel Alkalisierungsmittel, üblicherweise Alkali- oder Erdalkalihydroxide, Ammoniak oder organische Amine. Bevorzugte Alkalisierungsmittel sind Monoethanolamin, Monoisopropanolamin, 2-Amino-2-methyl-propanol, 2-Amino-2-methyl-1,3-propandiol, 2-Amino-2-ethyl-1,3-propandiol, 2-Amino-2-methylbutanol und Triethanolamin sowie Alkali- und Erdalkalimetallhydroxide. Insbesondere Monoethanolamin, Triethanolamin sowie 2-Amino-2-methyl-propanol und 2-Amino-2-methyl-1,3-propandiol sind im Rahmen dieser Gruppe bevorzugt. Auch die Verwendung von ω-Aminosäuren wie ω-Aminocapronsäure als Alkalisierungsmittel ist möglich.

Zur Ausbildung der eigentlichen Haarfarben durch einen oxidativen Prozess können übliche Oxidationsmittel, wie insbesondere Wasserstoffperoxid oder dessen Anlagerungsprodukte an Harnstoff, Melamin oder Natriumborat verwendet werden. Die Oxidation mit Luftsauerstoff als einzigem Oxidationsmittel kann allerdings bevorzugt sein. Weiterhin ist es möglich, die Oxidation mit Hilfe von Enzymen durchzuführen, wobei die Enzyme sowohl zur Erzeugung von oxidierenden Per-Verbindungen eingesetzt werden als auch zur Verstärkung der Wirkung einer geringen Menge vorhandener Oxidationsmittel. So können die Enzyme (Enzymklasse 1: Oxidoreduktasen) Elektronen aus geeigneten Entwicklerkomponenten (Reduktionsmittel) auf Luftsauerstoff übertragen. Bevorzugt sind dabei Oxidasen wie Tyrosinase und Laccase aber auch Glucoseoxidase, Uricase oder Pyruvatoxidase. Weiterhin sei das Vorgehen genannt, die Wirkung geringer Mengen (z. B. 1% und weniger, bezogen auf das gesamte Mittel) Wasserstoffperoxid durch Peroxidasen zu verstärken.

Zweckmäßigerweise wird die Zubereitung des Oxidationsmittels dann unmittelbar vor dem Färben der Haare mit der Zubereitung mit den Farbstoffvorprodukten vermischt. Das dabei entstehende gebrauchsfertige Haarfärbepräparat sollte bevorzugt einen pH-Wert im Bereich von 6 bis 10 aufweisen. Besonders bevorzugt ist die Anwendung der Haarfärbemittel in einem alkalischen Milieu. Die Anwendungstemperaturen können in einem Bereich zwischen 15 und 40 °C, bevorzugt bei der Temperatur der Kopfhaut, liegen. Nach einer Einwirkungszeit von ca. 5 bis 45, insbesondere 15 bis 30, Minuten wird das Haarfärbemittel durch Ausspülen von dem zu färbenden Haar entfernt. Das Nachwaschen mit einem Shampoo entfällt, wenn ein stark tensidhaltiger Träger, z. B. ein Färbeshampoo, verwendet wurde. Das Haar kann bevorzugt mit einem Mittel nachbehandelt werden, welches mindestens eine konditionierend wirkende Substanz enthält.

Insbesondere bei schwer färbbarem Haar kann die Zubereitung mit den Farbstoffvorprodukten ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

Unabhängig davon, welches der oben genannten Vorgehen im Rahmen des erfindungsgemäßen Verfahrens gewählt wird, kann die Ausbildung der Färbung dadurch unterstützt und gesteigert werden, dass dem Mittel bestimmte Metallionen zugesetzt werden. Solche Metallionen sind beispielsweise Zn²⁺, Cu²⁺, Fe²⁺, Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflusst werden.

Beispiele für erfindungsgemäß einsetzbare, strukturverbessernde Wirkstoffe stellen Vitamine und deren Derivate beziehungsweise Vorstufen dar. Erfindungsgemäß besonders bevorzugt sind Panthenol und seine physiologisch verträglichen Derivate. Solche Derivate sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 A1 offenbarten kationischen Panthenolderivate. Ein erfindungsgemäß bevorzugtes Panthenolderivat ist ferner dessen Vorstufe Pantolacton. Panthenol ist innerhalb dieser Gruppe bevorzugt. Ein weiteres Beispiel für ein strukturverbessemdes Vitamin ist Pyridoxin (Vitamin B6).

Weiterhin ist auch Polyvinylpyrrolidon (PVP) für seine faserstrukturverbessernden Eigenschaften bekannt und erfindungsgemäß bevorzugt.

Weitere, erfindungsgemäß besonders wirksame, strukturverbessernde Verbindungen stellen die Aldehyde dar. Besonders bevorzugte Beispiel sind Formaldehyd und Formaldehyd-abspaltende Verbindungen, wie beispielsweise Methoxymethylester, Dimethylol (thio) harnstoffderivate, Oxazolidinderivate, N-Hydroxymethylmaleinimid, Hexamethylentetramin und seine Derivate, Hydantoinderivate, Pyridinium-substituierte Dimethylether, Imidazolidinylharnstoff Derivate, Isothiazolinone, 2-Brom-2-nitropropan-diol und 5-Brom-5-nitro-1,3-dioxan. Weitere besonders bevorzugte Aldehyde sind Acetaldehyd, Glyoxal, Glycerinaldehyd und Glutardialdehyd.

Eine weitere geeignete Gruppe von strukturverbessernden Wirkstoffen sind Pflanzenextrakte.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß verwendbaren Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Erfindungsgemäß sind vor allem die Extrakte aus Eichenrinde, Brennessel, Hamamelis, Hopfen, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, grünem Tee, Ginseng und Ingwerwurzel bevorzugt.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2-80 Gew.-% Aktivsubstanz und als Lösungsmittel das bei ihrer Gewinnung eingesetzte Extraktionsmittel oder Extraktionsmittelgemisch.

Weiterhin kann es bevorzugt sein, in den erfindungsgemäßen Mitteln Mischungen aus mehreren, insbesondere aus zwei, verschiedenen Pflanzenextrakten einzusetzen.

Ebenfalls erfindungsgemäß als strukturverbessernde Wirkstoffe bevorzugt sind Honigextrakte. Diese Extrakte werden in analoger Weise zu den Pflanzenextrakten gewonnen und enthalten üblicherweise 1-10 Gew.-%, insbesondere 3-5 Gew.-%, Aktivsubstanz. Wasser/Propylenglykol-Mischungen können auch hier bevorzugte Extraktionsmittel sein.

Weitere strukturverbessernde Wirkstoffe sind Proteinhydrolysate, insbesondere Elastin-, Kollagen-, Keratin-, Milcheiweiß-, Sojaprotein-, Mandelprotein- und Weizenproteinhydrolysate, deren Kondensationsprodukte mit Fettsäuren sowie quaternisierte Proteinhydrolysate. Besonders bevorzugt sind stark abgebaute Keratinhydrolysate mit Molmassen im Bereich von 400 bis 800. Ferner sind quaternierte Proteinhydrolysate, wie sie beispielsweise unter den Handelsbezeichnungen Gluadin^{®} WQ (INCI-Bezeichnung: Laurdimonium Hydroxypropyl Hydrolyzed Wheat Protein) und Crotein^{®} Q (INCI-Bezeichnung: Hydroxypropyltrimonium Hydrolyzed Collagen) vertrieben werden, erfindungsgemäß besonders bevorzugt.

Neben den quaternierten Proteinhydrolysaten stellen auch quaternäre Polymere erfindungsgemäß bevorzugte strukturverbessernde Verbindungen dar. Besonders bevorzugt sind die Polymere, die unter den Handelsbezeichnungen Mirapol^{®} A15 (INCI-Bezeichnung: Polyquaternium-2), Onamer^{®} M (INCI-Bezeichnung; Polyquaternium-1) und Merquat^{®} 100 (INCI-Bezeichnung: Polyquatemium-6) vertrieben werden.

Ebenfalls faserstrukturverbessernde Wirkstoffe sind Mono-, Di- und Oligosaccharide wie beispielsweise Glucose, Galactose, Fructose, Fruchtzucker, Saccharose und Lactose. Weiterhin können auch Derivate dieser Pentosen und Hexosen, wie die entsprechenden On- und Uronsäuren (Zuckersäuren), Zuckeralkohole, Zuckeramine, wie beispielsweise N-Glucosamin, und Glykoside, erfindungsgemäß eingesetzt werden. Die Zuckersäuren können erfindungsgemäß in freier Form, in Form ihrer Salze, bevorzugt sind Calcium-, Magnesium- und Zink-Salze, und in Form ihrer Ester oder Lactone eingesetzt werden. Bevorzugte Zuckersäuren sind die Gluconsäure, Gluconsäure-γ-lacton, Lactobionsäure, die Glucuronsäure und ihre Mono- beziehungsweise Dilactone, die Pangaminsäure, die Zuckersäure, die Mannozuckersäure und ihre Mono- beziehungsweise Dilactone sowie die Schleimsäure und ihre Mono- beziehungsweise Dilactone. Bevorzugte Zuckeralkohole sind Sorbit, Mannit und Dulcit. Bevorzugte Glykoside sind die Methylglucoside. Glucose, N-Glucosamin und Gluconsäure sind aus dieser Gruppe besonders bevorzugt.

Auch gewisse Aminosäuren sind in Rahmen der vorliegenden Erfindung als haarstrukturverbessernde Wirkstoffe einsetzbar. Beispiele sind die in der DE-195 22 569, auf die hier ausdrücklich Bezug genommen wird, beschriebenen Aminosäuren Serin, Threonin und Tyrosin. Ferner sind auch Derivate des Serins, wie beispielsweise das Serinphosphat, erfindungsgemäß bevorzugt. Eine weitere strukturverbessernde Aminosäure stellt Lysin dar. Serin ist ein besonders bevorzugter faserstrukturverbessernder Wirkstoff.

Ebenfalls zur Strukturverbesserung können bestimmte Säuren, insbesondere α-Hydroxycarbonsäuren, und ihre Salze eingesetzt werden. Erfindungsgemäß bevorzugte strukturverbessernde Säuren sind Milchsäure, Äpfelsäure, Weinsäure, Glycerinsäure und Maleinsäure. Milchsäure ist besonders bevorzugt. Weiterhin verbessern spezielle Phosphonsäuren und ihre Salze die Struktur keratinischer Fasern. Erfindungsgemäß bevorzugte Phosphonsäuren sind die n-Octylphosphonsäure und die n-Decylphosphonsäure.

Weiterhin sind lipidlösliche Esteralkohole oder Esterpolyole für ihre strukturverbessernde Wirkung bekannt. Als lipidlöslich sind sie dann anzusehen, wenn sich 5 Gew.-% dieser Produkte in Cetylalkohol bei 80° C klar auflösen.

Besonders gut eignet sich dabei für die erfindungsgemäßen Haarbehandlungsmittel als Esterpolyol das Umsetzungsprodukt eines Pflanzenfettsäuremethylester-Epoxidats mit Trimethylpropan und mit einer Hydroxylzahl von 350 - 450. Ein solches Produkt auf Basis von Sojaölfettsäuremethylester-Epoxid und Trimethylolpropan ist unter der Handelsbezeichnung Sovermol^{®} 760 erhältlich.

Weiterhin kann als strukturverbessernder Wirkstoff Vitamin B₃ eingesetzt werden. Unter dieser Bezeichnung werden häufig die Verbindungen Nicotinsäure und Nicotinsäureamid (Niacinamid) geführt. Erfindungsgemäß bevorzugt ist das Nicotinsäureamid.

Auch Vitamin H ist als strukturverbessernder Wirkstoff im Sinne der vorliegenden Erfindung einsetzbar. Als Vitamin H wird die Verbindung (3a*S*,4*S*, 6a*R*)-2-Oxohexa-hydrothienol[3,4-*d*]-imidazol-4-valeriansäure bezeichnet, für die sich aber zwischenzeitlich der Trivialname Biotin durchgesetzt hat.

Erfindungsgemäß besonders bevorzugte strukturverbessernde Wirkstoffe sind ausgewählt aus Panthenol, physiologisch verträglichen Panthenol-Derivaten, Mono-, Di- und Oligosacchariden, Serin, Niacinamid, Polyvinylpyrrolidon, Gluconsäure, Biotin und den lipidlöslichen Esteralkoholen oder Esterpolyolen.

Die erfindungsgemäßen Mittel enthalten die strukturverbessernden Wirkstoffe bevorzugt in Mengen von 0,1 bis 5 Gew.-%, besonders bevorzugt in Mengen von 0,2 bis 2 Gew.-%.

In einer bevorzugten Ausführungsform der vorliegenden Erfindung enthalten die Mittel weiterhin eine Magnesiumverbindung. Die erfindungsgemäßen Mittel können durch Zugabe der Mg²⁺-Kationen hinsichtlich ihre strukturerhaltenden Eigenschaften weiter optimiert werden. Bevorzugte Magnesiumverbindungen sind anorganische und organische Mg²⁺-Salze, wie beispielsweise die Halogenide, die Carbonate und Hydrogencarbonate, das Acetat und das Citrat.

In einer weiteren bevorzugten Ausführungsform dieses Gegenstandes der Erfindung enthalten die Mittel mindestens ein Farbstoffvorprodukt vom Typ der Entwickler enthält. Hinsichtlich der bevorzugten Entwicklerkomponenten sei an dieser Stelle explizit auf die obigen Ausführungen verwiesen.

Ferner sind auch Mittel zur Färbung keratinischer Fasern Gegenstand der vorliegenden Erfindung, die unmittelbar vor der Anwendung durch Mischen einer ersten Komponente, enthaltend mindestens eine Entwicklerkomponente, mit einer zweiten Komponente, enthaltend mindestens ein Oxidationsmittel, erhalten werden und mindestens 1 Gew.-%, bezogen auf die Anwendungszubereitung, eines erfindungsgemäßen APG-Derivats enthalten.

Weiterhin können die Mittel der verschiedenen Gegenstände dieser Erfindung zur Nuancierung mindestens eine Kupplerkomponente enthalten. Auch an dieser Stelle wird explizit auf die obigen Ausführungen verwiesen.

Die erfindungsgemäßen Mittel können weiterhin alle für solche Zubereitungen bekannten Wirk-, Zusatz- und Hilfsstoffe enthalten. In vielen Fällen enthalten die Färbemittel mindestens ein weiteres Tensid, wobei prinzipiell sowohl anionische als auch zwitterionische, ampholytische, nichtionische und kationische Tenside geeignet sind. In vielen Fällen hat es sich aber als vorteilhaft erwiesen, die weiteren Tenside aus anionischen, zwitterionischen oder nichtionischen Tensiden auszuwählen.

Als weitere anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾ -oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammonium-glycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z.B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid^{®}S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex^{®} vertriebenen Methylhydroxyalkyldialkoyloxyalkyl-ammoniummethosulfate sowie die unter dem Warenzeichen Dehyquart^{®} vertriebenen Produkte wie Dehyquart^{®} AU-46.

Ein Beispiel für ein als kationisches Tensid einsetzbares quaternäres Zuckerderivat stellt das Handelsprodukt Glucquat^{®} 100 dar, gemäß INCI-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

In einer bevorzugten Ausführungsform enthält das erfindungsgemäße Mittel weitere Tenside ausgewählt aus zwitterionischen Tensiden, ampholytischen Tensiden, Dialkanolamiden, Aminoxiden und/oder Polyethylenglycolethern. Diese Komponenten können in dem erfindungsgemäßen Mittel beispielsweise in einer Menge von 0,1 bis 10 Gew.-%, bevorzugt in einer Menge von 0,1 bis 4,5 Gew.-%, bezogen auf das Mittel, enthalten sein.

Weiterhin können die erfindungsgemäßen Haarbehandlungsmittel bevorzugt noch einen konditionierenden Wirkstoff, ausgewählt aus der Gruppe, die von kationischen Tensiden, kationischen Polymeren, Alkylamidoaminen, Paraffinölen, pflanzlichen Ölen und synthetischen Ölen gebildet wird, enthalten.

Als konditionierende Wirkstoffe bevorzugt sein können kationische Polymere. Dies sind in der Regel Polymere, die ein quartäres Stickstoffatom, beispielsweise in Form einer Ammoniumgruppe, enthalten.
Bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate.
- polymere Dimethyldiallylammoniumsalze und deren Copolymere mit Acrylsäure sowie Estern und Amiden von Acrylsäure und Methacrylsäure. Die unter den Bezeichnungen Merquat^{®}100 (Poly(dimethyldiallylammoniumchlorid)), Merquat^{®}550 (Dimethyldiallylammoniumchlorid-Acrylamid-Copolymer) und Merquat^{®} 280 (Dimethyldiallylammoniumchlorid-Acrylsäure-Copolymer im Handel erhältlichen Produkte sind Beispiele für solche kationischen Polymere.
- Copolymere des Vinylpyrrolidons mit quaternierten Derivaten des Dialkylaminoacrylats und -methacrylats, wie beispielsweise mit Diethylsulfat quaternierte Vinylpyrrolidon-Dimethylaminomethacrylat-Copolymere. Solche Verbindungen sind unter den Bezeichnungen Gafquat^{®}734 und Gafquat^{®}755 im Handel erhältlich.
- Vinylpyrrolidon-Methoimidazoliniumchlorid-Copolymere, wie sie unter der Bezeichnung Luviquat^{®} angeboten werden.
- quaternierter Polyvinylalkohol
sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Besonders bevorzugt sind kationische Polymere der vier erstgenannten Gruppen, ganz besonders bevorzugt sind Polyquaternium-2, Polyquaternium-10 und Polyquaternium-22.

Als konditionierende Wirkstoffe weiterhin geeignet sind Silikonöle, insbesondere Dialkyl- und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190, DC 200, DC 344, DC 345 und DC 1401 vertriebenen Produkte sowie die Handelsprodukte Q2-7224 (Hersteller: Dow Corning; ein stabilisiertes Trimethylsilylamodimethicon), Dow Corning^{®} 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquatemäre Polydimethylsiloxane, Quaternium-80).

Ebenfalls einsetzbar als konditionierende Wirkstoffe sind Paraffinöle, synthetisch hergestellte oligomere Alkene sowie pflanzliche Öle wie Jojobaöl, Sonnenblumenöl, Orangenöl, Mandelöl, Weizenkeimöl und Pfirsichkernöl.

Gleichfalls geeignete haarkonditionierende Verbindungen sind Phospholipide, beispielsweise Sojalecithin, Ei-Lecithin und Kephaline.

Die in wässrigen Systemen, insbesondere in Färbecremes, zusätzlich einsetzbaren Alkohole beziehungsweise Alkoholgemische können geradkettig oder verzweigt sein und weisen 8 bis 22 Kohlenstoffatome auf. Sie können nativen Ursprungs oder synthetisch hergestellt sein. Vorzugsweise werden solche Alkohole oder Alkoholgemische eingesetzt, die in Kombination mit den anionischen Tensiden eine verdickende Wirkung zeigen. Die Alkohole sind in dem Mittel gemäß der vorliegenden Erfindung beispielsweise in einer Menge von 0,1 bis 10 Gew.-% und besonders bevorzugt in einer Menge von 1 bis 8 Gew.-%, bezogen auf das anwendungsfertige Mittel, enthalten.

Als weitere Komponente kann das erfindungsgemäße Mittel eine Fettsäure bzw. Fettsäureseife oder ein Fettsäuren- bzw. Fettsäureseifengemisch enthalten, wobei lineare oder verzweigte, gesättigte oder ungesättigte Fettsäuren bzw. Fettsäureseifen verwendet werden können. Diese können in Form der Säuren, die im Laufe des Herstellungsverfahrens neutralisiert werden, oder als neutralisierte Fettsäureseifen zugesetzt werden. Bevorzugt eingesetzte Fettsäuren sind Isostearinsäure, Ölsäure, Elaidinsäure oder deren Gemische bzw. deren Alkali- oder Ammoniumseifen. Die Fettsäure bzw. Fettsäureseife ist beispielsweise in einer Menge von 1 bis 5 Gew.-%, besonders bevorzugt in einer Menge von 1,5 bis 3,5 Gew.-%, bezogen auf das anwendungsfertige Mittel enthalten.

Zur Einstellung der Viskosität können zusätzlich übliche verdickende Additive in Mengen bis zu 10 Gew.-%, vorzugsweise von 0,1 bis 10 Gew.-% und besonders bevorzugt von 0,5 bis 5 Gew.-%, verwendet werden. Die Viskosität der anwendungsfertigen Mittel kann mit üblichen Standardmethoden (beispielsweise Brookfield-Viskosimeter RVD-VII bei 20 U/min und 20°C, Spindel 3) gemessen werden und liegt vorzugsweise im Bereich von 100 bis 5000 mPas. Bevorzugte flüssige bis gelförmige Mittel haben nach dieser Meßmethode Viskositäten von 200 bis 4000 mPas, wobei Werte zwischen 400 und 2000 mPas besonders bevorzugt sind.

Werden die Viskositäten mit einem Brookfield-Viskosimeter RVD-VII bei 4 U/min und 20°C mit Spindel 5) gemessen, liegen diese vorzugsweise im Bereich von 20.000 bis 100.000 mPas.

Geeignete Verdicker sind anorganische oder polymere organische Verbindungen. Es können auch Gemische aus mehreren Verdickern eingesetzt werden.

Zu den anorganischen Verdickern zählen beispielsweise Polykieselsäuren, Tonmineralien wie Montmorillonite, Zeolithe, Kieselsäuern und Tone, wie Bentonite.

Die organischen Verdicker stammen aus den Gruppen der natürlichen Polymere, der abgewandelten natürlichen Polymere und der vollsynthetischen Polymere.
Aus der Natur stammende Polymere, die als Verdickungsmittel Verwendung finden, sind beispielsweise Agar-Agar, Carrageen, Alginate, Xanthan-Gum, Karaya-Gummi, Tragant, Gummi arabicum, Alginate, Pektine, Polyosen, Guar-Mehl, Johannisbrotbaumkernmehl, Leinsamengummen, Dextrane, Stärke-Fraktionen und Derivate wie Amylose, Amylopektin und Dextrine, Gelatine und Casein.

Abgewandelte Naturstoffe stammen vor allem aus der Gruppe der modifizierten Stärken und Cellulosen, beispielhaft seien hier Carboxymethylcellulose und andere Celluloseether, wie zum Beispiel Hydroxyethyl- und Hydroxypropylcellulose, sowie Kernmehlether genannt.

Eine große Gruppe von Verdickungsmitteln, die breite Verwendung in den unterschiedlichsten Anwendungsgebieten finden, sind die vollsynthetischen Polymere wie Polyacryl- und Polymethacryl-Verbindungen, Vinylpolymere, wie Polyvinylalkohol, Polycarbonsäuren, Polyether, Polyimine, Polyamide und Polyurethane.

Als weitere Beispiele für vollsynthetische Polymere können auch anionische und kationische Polymere, vorzugsweise Copolymere aus anionischen und nichtionogenen Monomeren genannt werden, wobei solche, die aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt, bestehen, besonders bevorzugt sind.

Verdickungsmittel aus den genannten Substanzklassen sind kommerziell erhältlich und werden beispielsweise unter den Handelsnamen Acusol^{®}-820 (Methacrylsäure(stearylalkohol-20-EO)ester-Acrylsäure-Copolymer, 30%ig in Wasser, Rohm & Haas), Dapral^{®}-GT-282-S (Alkylpolyglykolether, Akzo), Deuterol^{®}-Polymer-11 (Dicarbonsäure-Copolymer, Schöner GmbH), Deuteron^{®}-XG (anionisches Heteropolysaccharid auf Basis von β-D-Glucose, D-Manose, D-Glucuronsäure, Schöner GmbH), Deuteron^{®}-XN (nichtionogenes Polysaccharid, Schöner GmbH), Dicrylan^{®}-Verdicker-O (Ethylenoxid-Addukt, 50%ig in Wasser/Isopropanol, Pfersse Chemie), EMA^{®}-81 und EMA^{®}-91 (Ethylen-Maleinsäureanhydrid-Copolymer, Monsanto), Verdicker-QR-1001 (Polyurethan-Emulsion, 19-21%ig in Wasser/Diglykolether, Rohm & Haas), Mirox^{®}-AM (anionische Acrylsäure-Acrylsäureester-Copolymer-Dispersion, 25%ig in Wasser, Stockhausen), SER-AD-FX-1100 (hydrophobes Urethanpolymer, Servo Delden), Shellflo^{®}-S (hochmolekulares Polysaccharid, mit Formaldehyd stabilisiert, Shell), Shellflo^{®}-XA (Xanthan-Biopolymer, mit Formaldehyd stabilisiert, Shell), Kelzan, Keltrol T (Kelco), Sepigel^{®} 305 (INCI-Bezeichnung: Polyacrylamid (and) C₁₃-C₁₄-Isoparaffin (and) Laureth-7) (SEPPIC) angeboten.

Eine weitere Erhöhung der Viskosität lässt sich auch durch Zugabe von Elektrolyten (Neutralsalzen) erreichen. Beispiele für derartige Salze sind die Alkalihalogenide, insbesondere Chloride, Alkalihydrogencarbonate, Alkalisulfate und Alkaliphosphate, wobei Natriumchlorid und Natriumsulfat besonders bevorzugt sind.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- zwitterionische und amphotere Polymere wie beispielsweise Acrylamidopropyl-trimethylammoniumchlorid/Acrylat-Copolymere und Octylacrylamid/Methylmethacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butyl-acrylamid-Terpolymere,
- Strukturanten wie Maleinsäure und Milchsäure,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genusssäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wasserstoffperoxid und andere Oxidationsmittel,
- Treibmittel wie Propan-Butan-Gemische, N₂O, Dimethylether, CO₂ und Luft,
- Antioxidantien.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Ein weiterer Gegenstand der vorliegenden Erfindung ist ein Verfahren zur oxidativen Färbung keratinischer Fasern, bei dem die Fasern mit einem Mittel gemäß Ansprunch 7 behandelt werden.

Dabei werden die Mittel beispielsweise aus einer W/O- oder O/W-Emulsion, enthaltend die Alkyl-Oligoglykosidcarboxylate mit einer Wasserstoffperoxid-Lösung unmittelbar vor der Anwendung zur oxidativen Färbung von Haaren gemischt.

Die folgenden Beispiele sollen den Gegenstand der vorliegenden Erfindung erläutern ohne ihn jedoch zu beschränken.

### Beispiele

### Beispiel 1 (oxidatives Färbemittel)

### a) Herstellung der ungleichmäßig strukturierten Testhaarsträhnen

Strähnen der Fa. Kerling (0,5 g Kerling, Naturweiß) wurden mittig abgebunden, und eine Hälfte wurde gebleicht (oberer Strähnenteil). Die andere Hälfte wurde zweimal gebleicht und zwei herkömmlichen Dauerwellbehandlungen mit dem Handelsprodukt Poly Lock-Normale Dauerwelle unterzogen (unterer Strähnenteil). Im Rahmen einer Dauerwellbehandlung wurden die Fasern jeweils in einem ersten Schritt für 30 Minuten bei Raumtemperatur der Reduktionslösung (enthaltend 7,9 Gew.-% Thioglykolsäure) ausgesetzt, mit reinem Wasser gespült und anschließend bei Raumtemperatur für 10 Minuten fixiert (Oxidationslösung, enthaltend 2,6 Gew.-% Wasserstoffperoxid). Nach der oxidativen Behandlung wurden die Fasern jeweils erneut gespült und getrocknet.

### b) Ausfärbungen

Zur Ausfärbung wurde folgendes Mittel verwendet:
Färbecreme:

| | Anmerkung | Mengen in Gew.-% |
|---|---|---|
| Hydrenol^{®} D | 1 | 8,1 |
| Lorol^{®} techn. | 2 | 2,7 |
| Texapon^{®} NSO | 3 | 3,5 |
| Plantapon^{®} LGC | 4 | 3,5 |
| Dehyton^{®} K | 5 | 5,0 |
| Eumulgin^{®} B 1 | 6 | 0,5 |
| Eumulgin^{®} B2 | 7 | 0,5 |
| Isostearinsäure | | 2,0 |
| Myristinsäure | | 0,5 |
| Carbopol^{®} ETD 2001 | 8 | 0,2 |
| KOH | | 0,6 |
| p-Toluylendiamin | | 1,43 |
| Resorcin | | 0,55 |
| m-Aminophenol | | 0,15 |
| 3-Amino-2-methylamino-6-methoxypridin | | 0,04 |
| Ammoniak (25 %ige, wässrige Lösung) | | 7,2 |
| Natriumsulfit | | 0,5 |
| Ammoniumsulfat | | 0,5 |
| Ascorbinsäure | | 0,4 |
| Turpinal^{®} SL | 9 | 0,2 |
| Natronwasserglas 40/20 | 10 | 0,5 |
| Propylenglykol | | 0,4 |
| Wasser | | ad 100 |

Entwicklerdispersion:

| | Anmerkung | Gew.-% |
|---|---|---|
| Dipicolinsäure | | 0,62 |
| Natriumpyrophosphat | | 0,03 |
| Turpinal^{®} SL | | 1,50 |
| Texapon^{®} NSO | | 2,00 |
| Dow Corning DB 110 A | 11 | 0,07 |
| Aculyn^{®} 33 A | 12 | 12,00 |
| Wasserstoffperoxid | | |
| (50 %ige wässrige Lösung) | | 12,0 |
| Wasser | | ad 100 |

Anmerkung:

| | |
|---|---|
| 1 | C₁₆-C₁₈-Fettalkohol (INCI-Bezeichnung: Cetearyl alcohol) |
| | (Cognis Deutschland) |
| 2 | C₁₂-C₁₈-Fettalkohol (INCI-Bezeichnung: Coconut Alcohol) |
| | (Cognis Deutschland) |
| 3 | Natriumlaurylethersulfat ( 27 % Aktivsubstanz INCI: Sodium Laureth Sulfate) |
| | (Cognis Deutschland) |
| 4 | Alkylpolyglucosid-Carboxylat Natriumsalz; 30 % Aktivsubstanz |
| | (erfindungsgemäß) (Natrium Carboxymethyl (C₁₀- bis C₁₆)-Alkyl |
| | Glucosid) (Cognis Deutschland) |
| 5 | N,N-Dimethyl-N-(C₈-C₁₈-kokosamidpropyl)-ammoniumacetobetain (ca. 30 % Aktivsubstanz; INCI-Bezeichnung: Aqua (Water), Cocamidopropyl Betaine) |
| | (Cognis Deutschland) |
| 6 | Cetylstearylakohol mit 12 EO-Einheiten (INCI-Bezeichnung: |
| | Ceteareth-12) (Cognis Deutschland) |
| 7 | Cetylstearylalkohol mit ca. 20 EO-Einheiten (INCI-Bezeichnung: |
| | Ceteareth-20) |
| 8 | Polyacrylsäure (INCI-Bezeichnung: Carbomer) (Noveon) |
| 9 | 1-Hydroxyethan-1,1-diphosphonsäure (INCI-Bezeichnung: |
| | Etidronic Acid, Aqua (Water)) (Solutia) |
| 10 | ca. 40 % Aktivsubstanz (INCI-Bezeichnung: Sodium Silicate) |
| | (Cognis Deutschland) |
| 11 | nichtionogene Silikonemulsion (10 Gew.-% Aktivsubstanz) |
| | (INCI-Bezeichnung: Dimethicone) (Dow Corning) |
| 12 | 30 Gew.-% Aktivsubstanz in Wasser (INCI-Bezeichnung: |
| | Acrylates Copolymer) (Rohm & Haas) |

50.0 g der Färbecreme wurden mit 50.0 g der Entwicklerdispersion vermischt. Die resultierende Anwendungsmischung wurde auf eine Testhaarsträhne, die gemäß 1.a) hergestellt wurde, aufgetragen. Nach 30 Minuten Einwirkungszeit bei 32 °C wurde das Haar gespült, mit einem üblichen Haarwaschmittel ausgewaschen und anschließend getrocknet.

Zu Vergleichszwecken wurde eine Färbecreme eingesetzt, die 7,0 Gew.-% Texapon^{®} NSO und kein Plantapon^{®} LGC enthielt.

Bei Verwendung der erfindungsgemäßen Färbecreme und der Vergleichscreme wurde jeweils zunächst eine gleichmäßige Färbung über die gesamte Strähne erhalten. Sodann wurden die Strähnen sechsmal mit einem herkömmlichen Shampoo gewaschen und anschließend getrocknet. Für die mit der Vergleichscreme gefärbte Strähne verlor die Färbung des unteren Strähnenteils sichtbar an Intensität, während der obere Strähnenteil nahezu seine Ausgangsfärbung behielt.

Die Strähne, die mit der erfindungsgemäßen Färbecreme behandelt wurde, behielt in beiden Strähnenteilen nahezu die Ausgangsfärbung. Damit wurde die Waschechtheit der Färbung auf strapaziertem Haar durch den Einsatz des erfindungsgemäßen APG-Carboxylats gegenüber der Vergleichsfärbecreme deutlich verbessert. Der Effekt war mit dem bloßen Auge zu erkennen.

## Patentansprüche

1. Verwendung von Alkyl-Oligoglykosidcarboxylaten die sich von Alkyl-Oligoglykosiden der allgemeinen Formel (I) ableiten,
R-O-(G)ₚ (I)
mit der Bedeutung
R C₆₋₂₂-Alkyl,
G Glykosideinheit, die sich von einem Zucker mit 5 oder 6 Kohlenstoffatomen ableitet,
p Zahl von 1 bis 10,
in Oxidationsmittel-haltigen Mitteln zur Behandlung keratinischer Fasern, zur Verbesserung der Farbstabilität der Ausfärbung in Mitteln zur Färbung von Haaren, insbesondere bei Anwendung auf strapaziertem Haar.

2. Verwendung nach Anspruch 1, **dadurch gekennzeichnet, dass** in den Alkyl-, Oligoglykosiden in mindestens einem der Reste G mindestens eine Hydroxylgruppe durch -O-C₁₋₁₂-Alkylen-COOM mit M H, Alkalimetall, NH₄ jersetzt ist.

3. Verwendung nach Anspruch 2, **dadurch gekennzeichnet, dass** sich in der allgemeinen Formel (I) die Glykosideinheit G von Aldosen oder Ketosen, insbesondere der Glucose ableitet, p eine Zahl von 1,1 bis 3,0 ist und R ein C₁₂₋₁₈-Alkylrest ist.

4. Verwendung nach einem der Ansprüche 2 oder 3, **dadurch gekennzeichnet, dass** ein Alkyloligoglykosidcarboxylat eingesetzt wird, in dem -O-C₁₋₁₂-Alkylen-COOM -O-(CH₂-)ₙCOOM mit M H, Na oder K und n =1, 2 oder 3 bedeutet.

5. Verwendung nach einem der Ansprüche 1 bis 4, **dadurch gekennzeichnet, dass** die Alkyl-Oligoglykosidcarboxylate in Kombination mit Wasserstoffperoxid und/oder dessen festen Anlagerungsprodukten verwendet werden.

6. Verwendung nach Anspruch 1 zur Verbesserung der Waschechtheit von gefärbtem strapaziertem Haar.

7. Mittel zur Behandlung keratinischer Fasern, insbesondere menschlicher Haare, enthaltend mindestens ein Alkyl-Oligoglykosidcarboxylat, wie es in einem der Ansprüche 1 bis 4 definiert ist, sowie mindestens ein Oxidationsmittel und mindestens ein Farbstoffvorprodukt vom Typ der Entwickler.

8. Mittel nach Anspruch 7, **dadurch gekennzeichnet, dass** es ein Mittel zur oxidativen Färbung von Haaren ist.

9. Mittel nach einem der Ansprüche 7 und 8, enthaltend 0,2 bis 15 Gew.-% der Alkyl-Oligoglykosidcarboxylate oder Gemische davon.

10. Mittel nach einen der Ansprüche 7 bis 9, **dadurch gekennzeichnet, dass** im Mittel Alkyl-Oligoglykosidcarboxylate oder Gemische davon und weitere anionische Tenside in einem Gewichtsverhältnis im Bereich von 1 : 0,002 bis 3,5 : 1 vorliegen.

11. Verfahren zur oxidativen Färbung keratinischer Fasern, **dadurch gekennzeichnet, dass** die Fasern mit einem Mittel nach einem der Ansprüche 7 bis 10 behandelt werden.

12. Verfahren nach Anspruch 10 oder 11, **dadurch gekennzeichnet, dass** die. Mittel aus einer W/O- oder O/W-Emulsion, enthaltend die Alkyl-Oligoglykosidcarboxylate nach einem der Anspruche 1 bis 5 und eine Wasserstoffperoxid-Lösung unmittelbar vor der Anwendung zur oxidativen Färbung von Haaren gemischt werden.

## Claims

1. Use of alkyl-oligoglycoside carboxylates, which are derived from alkyl-oligoglycosides of general formula (I),
R-O-(G)ₚ (I)
with the meaning
R is a C₆-C₂₂ alkyl
G is a glycoside unit, which is derived from a sugar with 5 or 6 carbon atoms,
p is a number from 1 to 10,
in oxidant-containing agents for treating keratinous fibers, in particular for hair treatment for improving color stability of the coloration in agents for dyeing hair, in particular in the application on damaged hair.

2. The use according to claim 1, **characterized in that** in the alkyl-oligoglycosides, in at least one of the radicals G, at least one hydroxyl group is replaced with -O-(C₁-C₁₂ alkylen)-COOM with M being H, an alkaline metal, NH₄.

3. The use according to claim 2, **characterized in that** in the general formula (I), the glycoside unit G is derived from aldoses or ketoses, in particular glucose, p is a number from 1.1 to 3.0 and R is a C₁₂-C₁₈ alkyl radical.

4. The use according to any of claims 2 or 3, **characterized in that** an alkyl-oligoglycoside carboxylate is applied, in which -O-(C₁-C₁₂ alkylene)-COOM means -O-(CH₂)ₙCOOM with M being H, Na or K, and n = 1, 2 or 3.

5. The use according to any of claims 1 to 4, **characterized in that** the alkyl-oligoglycoside carboxylates are used in combination with hydrogen peroxide and/or its solid addition products.

6. The use according to claim 1 for improving fastness to washing of dyed damaged hair.

7. An agent for treating keratinous fibers, in particular human hair, containing at least one alkyl-oligoglycoside carboxylate as defined in any of claims 1 to 4, as well as at least one oxidant and at least one coloring agent precursor product of the developer type.

8. The agent according to claim 7, **characterized in that** it is an agent for oxidative dyeing of hair.

9. The agent according to any of claims 7 and 8, containing 0.2 to 15% by weight of the alkyl-oligoglycoside carboxylates or mixtures thereof.

10. The agent according to any of claims 7 to 9, **characterized in that** alkyl-oligoglycoside carboxylates or mixtures thereof and further anionic surfactants are present in the agent in a weight ratio in the range from 1:0.002 to 3.5:1.

11. A method for oxidative dyeing of keratinous fibers, **characterized in that** the fibers are treated with an agent according to any of claims 7 to 10.

12. The method according to claim 11, **characterized in that** the agents consisting of a W/O or O/W emulsion containing the alkyl-oligoglycoside carboxylates according to any of claims 1 to 5 and a hydrogen peroxide solution are directly mixed before the application for oxidative dyeing of hair.

## Revendications

1. Utilisation de carboxylates d'alkyloligoglycosides qui dérivent d'alkyloligoglycosides répondant à la formule générale (I) :
R-O-(G)ₚ (I)
dans laquelle
R représente un groupe alkyle en C₆-C₂₂,
G représente une unité glycoside qui dérive d'un sucre contenant 5 ou 6 atomes de carbone ;
p représente un nombre de 1 à 10 ;
dans des agents contenant des agents d'oxydation pour le traitement de fibres kératiniques, en particulier pour le traitement des cheveux, pour améliorer la solidité de la coloration dans des agents pour la teinture des cheveux, en particulier lors d'une utilisation sur des cheveux abîmés.

2. Utilisation selon la revendication 1, **caractérisée en ce que**, dans les alkyloligoglycosides, dans au moins un des radicaux G, au moins un groupe hydroxyle est remplacé par un groupe -O-alcylène(en C₁-C₁₂)-COOM où M représente un atome d'hydrogène, un métal alcalin, un groupe NH₄.

3. Utilisation selon la revendication 2, **caractérisée en ce que**, dans la formule générale (I), l'unité glycoside G dérive d'aldoses ou de cétoses, en particulier du glucose, p représente un nombre de 1,1 à 3,0 et R représente un radical alkyle en C₁₂-C₁₈.

4. Utilisation selon l'une quelconque des revendications 2 ou 3, **caractérisée en ce qu'**on met en oeuvre un carboxylate d'alkyloligoglycoside dans lequel groupe le -O-alkylène(en C₁-C₁₂)COOM représente un groupe -O-(CH₂)ₙCOOM où M représente un atome d'hydrogène, un atome de sodium ou un atome de potassium et n = 1, 2 ou 3.

5. Utilisation selon l'une quelconque des revendications 1 à 4, **caractérisée en ce qu'**on utilise les carboxylates d'alkyloligoglycosides en combinaison avec du peroxyde d'hydrogène et/ou ses produits d'addition solides.

6. Utilisation selon la revendication 1, pour améliorer la résistance au lavage de cheveux teints abîmés.

7. Agent pour le traitement de fibres kératiniques, en particulier de cheveux humains, contenant au moins un carboxylate d'alkyloligoglycoside, tel qu'il est défini dans l'une quelconque des revendications 1 à 4, et au moins un agent d'oxydation et au moins un précurseur de colorant du type des développeurs.

8. Agent selon la revendication 7, **caractérisé en ce qu'**il s'agit d'un agent pour la la teinture des cheveux par oxydation.

9. Agent selon l'une quelconque des revendications 7 et 8, contenant des carboxylates d'alkyloligoglycosides ou leurs mélanges à concurrence de 0,2 à 15 % en poids.

10. Agent selon l'une quelconque des revendications 7 à 9, **caractérisé en ce que**, sont présents, dans l'agent, des carboxylates d'alkyloligoglycosides ou leurs mélanges et d'autres agents tensioactifs anioniques dans un rapport pondéral qui se situe dans la plage de 1 : 0,002 à 3,5 : 1.

11. Procédé pour la teinture de fibres kératiniques par oxydation, **caractérisé en ce qu'**on traite les fibres avec un agent selon l'une quelconque des revendications 7 à 10.

12. Procédé selon la revendication 11, **caractérisé en ce qu'**on mélange les agents constitués d'une émulsion du type eau/huile ou d'une émulsion huile/eau, contenant les carboxylates d'alkyloligoglycosides selon l'une quelconque des revendications 1 à 5, et une solution de peroxyde d'hydrogène, directement avant l'utilisation pour la teinture de cheveux par oxydation.
